(19) Europäisches Patentamt
European Patent Office
Office européen des brevets

(11) EP 2 034 949 B2

(12) # NEUE EUROPÄISCHE PATENTSCHRIFT
Nach dem Einspruchsverfahren

(45) Veröffentlichungstag und Bekanntmachung des Hinweises auf die Entscheidung über den Einspruch:
**28.06.2023 Patentblatt 2023/26**

(45) Hinweis auf die Patenterteilung:
**17.04.2013 Patentblatt 2013/16**

(21) Anmeldenummer: **07730186.9**

(22) Anmeldetag: **15.06.2007**

(51) Internationale Patentklassifikation (IPC):
***A61K 8/35*** (2006.01) ***A61K 8/40*** (2006.01)
***A61K 8/41*** (2006.01) ***A61K 8/49*** (2006.01)
***A61Q 17/04*** (2006.01)

(52) Gemeinsame Patentklassifikation (CPC):
**A61Q 17/04; A61K 8/35; A61K 8/4966; A61P 17/16; A61P 43/00**

(86) Internationale Anmeldenummer:
**PCT/EP2007/055944**

(87) Internationale Veröffentlichungsnummer:
**WO 2007/147785 (27.12.2007 Gazette 2007/52)**

(54) **VERFAHREN ZUR ERHÖHUNG DES LICHTSCHUTZFAKTORS EINER KOSMETISCHEN UND/ ODER DERMATOLOGISCHEN ZUBEREITUNG**

METHOD FOR INCREASING THE SUN PROTECTION FACTOR OF A COSMETIC AND/OR DERMATOLOGICAL PREPARATION

PROCÉDÉ D'AUGMENTATION DU COEFFICIENT DE PROTECTION SOLAIRE D'UNE PRÉPARATION COSMÉTIQUE ET/OU DERMATOLOGIQUE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **23.06.2006 EP 06115950**

(43) Veröffentlichungstag der Anmeldung:
**18.03.2009 Patentblatt 2009/12**

(73) Patentinhaber: **BASF SE**
**67056 Ludwigshafen am Rhein (DE)**

(72) Erfinder: **FLÖSSER-MÜLLER, Heike**
**67061 Ludwigshafen (DE)**

(74) Vertreter: **BASF IP Association**
**BASF SE**
**GBI-C006**
**67056 Ludwigshafen (DE)**

(56) Entgegenhaltungen:
**EP-A2- 1 046 391** **WO-A-2005/094772**
**WO-A1-03/039507** **DE-A1- 10 143 960**
**DE-A1- 10 143 962** **DE-A1- 10 229 526**
**DE-A1- 10 328 547** **DE-A1-102004 003 478**

- **WUENSCH T: "Synergistic effects with high performance UV-Filters" IFSCC INTERNATIONAL CONFERENCE, XX, XX, Bd. 21st, 2000, Seiten 530-535, XP009089926 in der Anmeldung erwähnt**
- **Presseinformation BASF: Innovativer UVA-I-Filter der BASF AG zugelassen,veröffentlicht 31.01.2005**
- **XXIst IFSCC International Congress 2000 Berlin, Proceedings 2000, S.530-535:Synergistic effects with high performance UV Filters**
- **Cosmetic Solutions: Uvinul® A Plus, Broschure der BASF, Illustration der Produkteigenschaft von Uvinul® A Plus (UV-Spektrum, in vivo SPF)**
- **N.Lowe,, N.Shaat, M.Pathak; Marcel Dekker: Sunscreens, Development, Evaluation and Regulatory Aspects; Second Edition (1997), S.382-384**
- **Brazilian Journal of Pharmaceutical Sciences, vol.40, nr.3, jul./sept., 2004, S 381-385**
- **Cosmetic Science Technology: In silico determination of Topical Sun protection (2011)**
- **Journal of Cosmetic Science, 53,11-26 (2002): Prediction of sun protection factors by calculation of transmissions with a calibrated step film model**
- **Journal of Pharmaceutical Sciences, Vol 93, No 7, (2004): Prediction of Sun Protection Factors and UVA Parameters of Sunscreens by Using a Calibrated Step Film Model(2004)**

- **Commission Recommendation of 22 September 2006 on the efficacy of sunscreen products and the claims made relating thereto**

Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft ein Verfahren zur Erhöhung des Lichtschutzfaktors einer kosmetischen und/oder dermatologischen Zubereitung, die Verwendung von UV-A-Filtern zur Erhöhung des Lichtschutzfaktors einer solchen Zubereitung und spezielle lichtschutzwirksame kosmetische und/oder dermatologische Zubereitungen.

Die in kosmetischen und dermatologischen Zubereitungen eingesetzten Lichtschutzmittel haben die Aufgabe, die schädigenden Wirkungen des Sonnenlichtes, das heißt insbesondere die der UV-Strahlung des Sonnenlichtes, auf die menschliche Haut zu verringern (Chemie in unserer Zeit, 2004, 38, 98-112). Daneben dienen diese Lichtschutzmittel aber auch dem Schutz von Inhaltsstoffen der Formulierung vor Zerstörung oder Abbau durch UV-Strahlung. Die Verwendung von Lichtschutzfiltersubstanzen in kosmetischen Mitteln für den Schutz der menschlichen Haut ist in den meisten Industrieländern durch Positivlisten mit Maximalwerten für den Einsatz solcher Substanzen gesetzlich geregelt.

UV-Strahlung wird, je nach Wellenlänge, in UV-A-Licht (320-400 nm) und UV-B-Licht (280-320 nm) und UV-C-Licht (100-280 nm) unterteilt.

Strahlen mit einer Wellenlänge, die kleiner als 290 nm ist, werden von der Ozonschicht in der Erdatmosphäre absorbiert und kommen auf der Erde nicht an. UV-Strahlen im Bereich zwischen 290 nm und 320 nm, sie zählen zum sogenannten UV-B-Bereich, können auf der Haut z. B. ein Erythem, d.h. einen Sonnenbrand, oder sogar mehr oder weniger starke Verbrennungen hervorrufen. Als ein Maximum der Erythemwirksamkeit des Sonnenlichtes wird der engere Bereich um 308 nm angegeben.

Der Lichtschutzfaktor (LSF, oft auch, dem englischen Sprachgebrauch angepasst, SPF = Sun Protection Factor, genannt) gibt an, um wie viel länger die mit dem Lichtschutzmittel geschützte Haut der Sonnenstrahlung ausgesetzt werden kann, bis die gleiche Erythemreaktion auftritt wie bei der ungeschützten Haut (also zehnmal solang gegenüber ungeschützter Haut bei LSF = 10). Der Lichtschutzfaktor ist also insbesondere ein Maß für den Schutz gegen UV-B-Strahlung. Für die Vermarktung von Sonnenschutzprodukten ist die Angabe des SPF in vivo, also gemessen am Menschen, verpflichtend. In Europa, Südamerika und Japan regelt das Protokoll der "International SPF Test Method 2003" die Bestimmung des SPF in vivo.

Zum Schutz gegen UV-B-Strahlung sind zahlreiche Verbindungen bekannt, bei denen es sich u.a. um Triazinderivate; Derivate des 3-Benzylidencamphers, der 4-Aminobenzoesäure, der Zimtsäure, der Salicylsäure, des Benzophenons sowie des 2-Phenylbenzimidazols handelt.

Auch die UV-A-Strahlung, der Bereich zwischen 320 nm und 400 nm, hat hautschädigende, aber daneben auch produktschädigende Wirkung, und somit ist es ebenfalls wichtig, UV-A Filtersubsubstanzen in kosmetischen und dermatologischen Formulierungen zur Verfügung zu haben. Man hat lange Zeit fälschlicherweise angenommen, dass die langwellige UV-A-Strahlung nur eine vernachlässigbare biologische Wirkung aufweist. Im Gegensatz zu UV-B-Licht, welches nur bis in die Epidermis (Oberhaut) eindringt, gelangt UV-A Strahlung bis in die tiefen Schichten der Dermis (Lederhaut), wo sich z. B. auch die Blutgefäße befinden. Ferner ist UV-A Licht, ebenfalls im Gegensatz zu UV-B Licht, in der Lage, Glasfenster und z. B. Autoscheiben zu durchdringen, sodass der Mensch auch innerhalb von Gebäuden oder im Auto nicht geschützt ist. Inzwischen ist durch zahlreiche Studien belegt, dass UV-A-Strahlung im Hinblick auf die Auslösung photodynamischer, speziell photoallergischer und phototoxischer Reaktionen und chronischer Veränderungen der Haut mindestens ebenso gefährlich wie die UV-B-Strahlung ist.

So ist es u.a. erwiesen, dass selbst kleine aber regelmäßige Dosen an UV-A-Strahlung, wie sie unter ganz normalen Alltagsbedingungen vorkommen, ausreichen, um z. B. das Bindegewebe der Haut, genauer gesagt die Collagen- und Elastinfasern signifikant zu schädigen. Diese Hautschädigung ist, zusammen mit weiteren chronischen lichtbedingten Hautveränderungen allgemein bekannt unter dem Begriff der "vorzeitigen Hautalterung" oder "Photoaging". Zum klinischen Erscheinungsbild der durch Licht vorzeitig gealterten Haut gehören beispielsweise Falten und Fältchen, eine verringerte Elastizität und Festigkeit sowie verringerte Feuchtigkeit. Ferner können die von lichtbedingter Hautalterung betroffenen Partien eine unregelmäßige Pigmentierung (z. B. "Altersflecken") und Keratosen aufweisen.

UV-A-Strahlung ist weiterhin in der Lage, die DNA zu schädigen, was im schlimmsten Fall zu Hautkrebs führen kann.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung weitgehend unabhängig von jahres- und tageszeitlichen oder geographischen Faktoren Tag für Tag relativ konstant. Es ist daher besonders wichtig, nicht nur in reinen Sonnenschutzpräparaten UV-A Filter zu verwenden, sondern auch in der Tagespflege, wie auch in der dekorativen Kosmetik, um die Haut vor chronischen Hautschäden, die aus der schwachen aber regelmäßigen UV-A Exposition resultieren, zu schützen und z. B. der vorzeitigen Hautalterung entgegenzuwirken.

Im allgemeinen ist das Lichtabsorptionsverhalten von Lichtschutzfiltersubstanzen sehr gut bekannt und dokumentiert. Für die Dosierung der Substanzen in den fertigen Formulierungen können die Absorptionsspektren allein allenfalls eine Orientierungshilfe bieten. Wechselwirkungen der UV-Filtersubstanzen untereinander, mit Inhaltsstoffen der Formulierung oder mit der Haut selbst können unvorhersehbare Effekte auf die Schutzwirkung haben. Ferner ist es

in der Regel schwierig vorab abzuschätzen, wie gleichmäßig und in welcher Schichtdicke die Filtersubstanzen in und auf der Hornschicht der Haut verteilt sind. All dies hat also Auswirkungen auf die Lichtschutzwirkung der Präparate und macht eine exakte Vorhersage schwierig bzw. unmöglich.

Zur Prüfung der UV-A-Schutzleistung gibt es für die meisten Länder bisher noch keine harmonisierte und allgemein bindende Methode. Üblicherweise wird jedoch die PPD-Methode verwendet (PPD = persistent pigment darkening). Diese ist die gesetzliche Basis in Japan (Japan Cosmetic Industry Association (JCIA); Measurement standard for UVA protection efficacy. Jan. 1, 1996.). Hierbei wird - ähnlich der Bestimmung des Lichtschutzfaktors - ein Wert ermittelt, der angibt, um wie viel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit verwendete und etablierte Klassifizierung der UV-A Schutzleistung einer Sonnenschutzformulierung ist der sogenannte Australische Standard (AS/NZS 2604:1997). Dabei wird die Transmission (= Durchlässigkeit) der Zubereitung im UV-A-Bereich gemessen. Um den Standard zu erfüllen, darf die Zubereitung maximal 10 % der einfallenden UV-A-Strahlung im Bereich 320 bis 360 nm durchlassen, muss also mind. 90 % der Strahlung in diesem Bereich blockieren.

In der Vergangenheit lag das Hauptaugenmerk im Sonnenschutz auf der Vermeidung von Sonnenbrand, das heißt dem Schutz vor erythemwirksamer UV-B Strahlung, ausgedrückt in immer höheren SPF Werten, sogar bis SPF > 100. Häufig lag bei Produkten der Vergangenheit kein oder nur unzureichender UV-A Schutz in diesen Produkten vor.

Mit dem zunehmenden Wissen über die schädigende Wirkung von UV-A-Strahlung wird auch die Notwendigkeit für eine verstärkte Verwendung von UV-A-Filtern für den Hautschutz offensichtlich und eine Reglementierung für einen Mindest-UV-A Schutz in Sonnenschutzprodukten erforderlich. Dies wird durch die derzeitigen verstärkten Aktivitäten zum Thema Mindestmaß an UV-A-Schutz und Festlegung einer harmonisierten UV-A-Bestimmungsmethode in Sonnenschutzmitteln von Seiten der Industrie (COLI-PA) als auch der EU Kommission widergespiegelt.

Zum Schutz gegen UV-A-Strahlen werden häufig Derivate des Dibenzoylmethans verwendet, deren Photostabilität jedoch nicht in ausreichendem Maße gegeben ist (Int. J. Cosm. Science 10, 53, 1988).

EP-A-1 046 391 beschreibt die Verwendung von aminosubstituierten Hydroxybenzophenonen als photostabile UV-A-Filter in kosmetischen Zubereitungen.

Da die Erythemwirksamkeit von UV-B-Licht bis zu 1000 mal stärker ist als die von UV-A-Licht, und der Lichtschutzfaktor (LSF) das Maß für den Schutz vor Erythem angibt, wird in einer Sonnenschutzformulierung zur Erhöhung des Schutzes vor Sonnenbrand und damit zur Erhöhung des Lichtschutzfaktors üblicherweise die Menge an Substanzen, die im UV-B-Bereich ihr Absorptionsmaximum aufweisen (UV-B-Filter), erhöht.

UV-A-Filter hingegen werden vorwiegend zum Schutz vor den oben genannten Folgen des UV-A-Lichtes eingesetzt.

Unter dem Begriff der UV-Schutzleistung wird sowohl die Schutzleistung gegenüber UV-A-Strahlung als auch gegenüber UV-B-Strahlung verstanden.

In WO 2005/094772 wird beispielsweise eine Tagescreme beschrieben, die als einzigen UV-Filter 2-(4-Diethylamino-2-hydroxybenzoyl)benzoesäurehexylester enthält, der ein photostabiler UV-A-Filter ist. Die beschriebene Tagescreme reduziert die Bräunung der Haut durch Sonnenbestrahlung. Lichtschutzfaktoren werden nicht angegeben.

In EP-A-1 290 999 werden kosmetische Stifte mit sehr guten Eigenschaften hinsichtlich Klebrigkeit, Hautverträglichkeit und Hautpflegeleistungen beschrieben, die neben de UV-A-Filter 2-(4-Diethylamino-2-hydroxybenzoyl)benzoesäurehexylester weitere UV-Filtersubstanzen enthalten. In Beispiel 4 wird eine Stiftformulierung beschrieben, die einen höheren Anteil an UV-A-Filter als die Summe an UV-B- und/oder Breitbandfiltern enthält, während in den übrigen Formulierungen jeweils die Summe der Gewichtsanteile an UV-A-Filtern geringer ist als die Summe der Gewichtsanteile an UV-B- und/oder Breitbandfiltern. Lichtschutzfaktoren werden nicht angegeben.

In den Proceedings zum 21st IFSCC International Congress 2000, Berlin, Seite 530 ff, wird ein positiver Effekt beschrieben, den die Zugabe kleiner Mengen an einem UV-A-Filter (BMDBM) auf den LSF bewirkt. Das Massenverhältnis der UV-A-Filter zu dem der verwendeten UV-B-Filter war dabei deutlich kleiner als 0,5.

In WO 03/039507 werden Lichtschutzformulierungen beschrieben, die als UV-A-Filter insbesondere 2-(4-Diethylamino-2-hydroxybenzoyl)benzoesäurehexylester und als UV-B-und/oder Breitband-Filter mindestens ein schwerlösliches Triazin- und/oder Benzotriazolderivat enthalten. Für die verschiedenen Formulierungen werden keine Werte für Lichtschutzfaktoren angegeben.

Es besteht weiterhin Bedarf an immer wirksameren Sonnenschutzformulierungen, die sowohl die Auswirkungen der Sonnenbrand auslösende UV-B-Strahlung als auch die Auswirkungen der UV-A-Strahlung, die unter anderem die vorzeitige Hautalterung ("photoaging") beschleunigt, reduzieren. Dabei soll eine möglichst effiziente Ausnutzung der zur Verfügung stehenden Filtersubstanzen erzielt werden, also eine hohe zotriazolderivat enthalten. Für die verschiedenen Formulierungen werden keine Werte für Lichtschutzfaktoren angegeben.

Es besteht weiterhin Bedarf an immer wirksameren Sonnenschutzformulierungen, die sowohl die Auswirkungen der Sonnenbrand auslösende UV-B-Strahlung als auch die Auswirkungen der UV-A-Strahlung, die unter anderem die vorzeitige Hautalterung ("photoaging") beschleunigt, reduzieren. Dabei soll eine möglichst effiziente Ausnutzung der

zur Verfügung stehenden Filtersubstanzen erzielt werden, also eine hohe Sonnenschutzwirkung mit einer möglichst geringen Menge an Lichtschutzfiltersubstanz, um eine unnötige Belastung insbesondere der Haut, aber auch der Umwelt mit Chemikalien zu vermeiden. Außerdem erhöht ein geringerer Masseanteil an effizienten UV-Filtern die Flexibilität des Formulierers, z. B. größere oder andere Anteile kosmetischer Ingredienzien zu verwenden. Weiterhin ist es wünschenswert, mit einer geringen Anzahl von Lichtschutzfiltersubstanzes in einer Formulierung die gewünschte Wirkung zu erreichen. Dies reduziert die Komplexität einer kosmetischen bzw. dermatologischen Formulierung.

Ziel ist es also, mit möglichst wenig UV-Filtersubstanz sowohl einen möglichst hohen UV-A- als auch hohen UV-B-Schutz zu erreichen.

Es war Aufgabe der vorliegenden Erfindung, Möglichkeiten aufzuzeigen, wie insbesondere der Erythemschutz (ausgedrückt als LSF oder SPF) unter effizienter Ausnutzung der eingesetzten Lichtschutzfiltersubstanzen erhöht werden kann und gleichzeitig ein sehr guter UV-A-Schutz erreicht wird.

Diese Aufgabe wird durch ein Verfahren zur Erhöhung des Lichtschutzfaktors einer kosmetischen und/oder dermatologischen Zubereitung, die mindestens einen UV-B-Filter, ausgewählt aus Ethylhexyl Triazon und Diethylhexyl Butamido Triazon, und optional UV-Breitband-Filter enthält, durch Zugabe eines UV-A-Filters während der Herstellung der Zubereitung gelöst, dadurch gekennzeichnet, dass in der fertigen kosmetischen und/oder dermatologischen Zubereitung ein Wert für das Verhältnis der Summe der Masse der UV-A-Filter zur Summe der Masse der UV-B-Filter und Breitband-Filter von 1 bis 6 eingestellt wird, dadurch gekennzeichnet, dass 2-(4-Diethylamino-2-hydroxybenzoyl)-benzoesäurehexylester als einziger UV-A-Filter eingesetzt wird. Die Erfindung beinhaltet auch die Verwendung gemäß Anspruch 2 und die Zubereitung gemäß Anspruch 3.

Der Lichtschutzfaktor (LSF, oft auch, dem englischen Sprachgebrauch angepasst, SPF = Sun Protection Factor genannt) ist wie voranstehend erläutert ein Maß für den Schutz gegen UV-B-Strahlung.

Erhöhung des Lichtschutzfaktors einer kosmetischen und/oder dermatologischen Zubereitung bedeutet erfindungsgemäß die Erzielung eines höheren LSF bzw. im englischen Sprachraum des SPF bestimmt durch die dem Fachmann bekannte Internationalen SPF Methode (2003), um wenigstens 1 bis 3, bevorzugt um wenigstens 4 bis 6, insbesondere um wenigstens 7 bis 9 SPF Einheiten, je nach Ausgangswert und Menge an zugegebenem UV-A Filter, durch das erfindungsgemäße Verfahren.

Bevorzugt wird in dem erfindungsgemäßen Verfahren eine Erhöhung um mindestens 1, bevorzugt mindestens 1,3 SPF-Einheiten bei konstant gehaltener Summe der Gewichtsanteile der UV-B-Filter und/oder Breitband-Filter in der Formulierung durch Erhöhung des UV-A-Filters um 1 Gew.-% bezogen auf die Masse der fertigen Zubereitung erreicht.

Im allgemeinen werden unter kosmetischen und/oder dermatologischen Zubereitungen Mischungen oder Formulierungen verstanden, die zur topischen Anwendung auf Haut oder Haar eingesetzt werden und die geeignet sind (i) zur Prävention von Schädigungen der menschlichen Haut und/oder menschlicher Haare, (ii) zur Behandlung von bereits aufgetretenen Schädigungen der menschlichen Haut und/oder menschlicher Haare, (iii) zur Pflege der menschlichen Haut und/oder menschlicher Haare und/oder (iv) zur Verbesserung des Hautgefühls (sensorische Eigenschaften). Explizit umfasst sind Mittel zur dekorativen Kosmetik. Bei den in dem erfindungsgemäßen Verfahren beschriebenen kosmetischen und/oder dermatologischen Zubereitungen handelt es sich um Präparate, deren Hauptindikation vor allem (z. B. bei Sonnenschutzpräparaten) oder aber unter anderem (z. B. bei Tagespflegeprodukten, bei Anti-Aging-Produkten, bei Selbstbräunungspräparaten) den Schutz der Haut vor Schädigungen durch Sonnenlicht, im speziellen durch UV-B- (280 bis 320 nm) und UV-A-Strahlung (>320 nm) ist. Die kosmetischen und/oder dermatologischen Zubereitungen enthalten neben den UV-Filtersubstanzen in einem kosmetisch verträglichen Medium geeignete Hilfs- und Zusatzstoffe, welche im Hinblick auf das spezielle Anwendungsgebiet gewählt werden. Derartige Hilfs- und Zusatzstoffe sind dem Fachmann geläufig und können z.B. Handbüchern der Kosmetik, beispielsweise Schrader, Grundlagen und Rezepturen der Kosmetika, Hüthig Verlag, Heidelberg, 1989, ISBN 3-7785-1491-1, oder Umbach, Kosmetik: Entwicklung, Herstellung und Anwendung kosmetischer Mittel, 2. erweiterte Auflage, 1995, Georg Thieme Verlag, ISBN 3 13 712602 9, entnommen werden.

Unter dem Begriff UV-A-Filter werden insbesondere öllösliche oder wasserlösliche Substanzen, aber auch schwerlösliche oder pigmentartige Substanzen verstanden, deren Absorptionsmaximum im UV-A-Bereich liegt, also im Bereich zwischen 320 und 400 nm.

Der erfindungsgemäss verwendete UV-A-Filter weist die folgende Struktur auf:

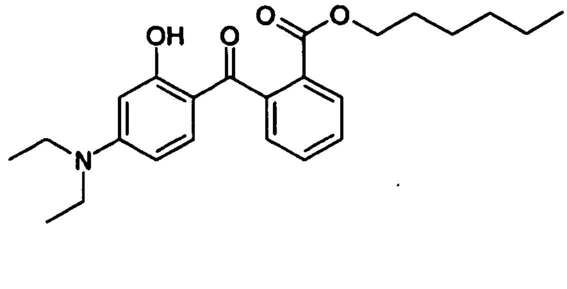

Diese Verbindung wird unter dem Namen Uvinul® A Plus von der BASF AG vertrieben.

Unter einem photostabilen UV-A-Filter versteht man eine Filtersubstanz, die unter realistischen Bestrahlungsbedingungen ihre Absorptionsfähigkeit nicht oder nur wenig verliert (z. B. bis zu 10 % Verlust nach sonnensimulierter Bestrahlung über 10 Standard-MED).

Unter MED versteht man die minimale erythemwirksame Strahlungsdosis, d. h. die Bestrahlungsdosis, die bei der ungeschützten Haut erste Zeichen eines Erythems, das heißt ein leichte Rötung hervorruft. Die MED ist individuell verschieden. Als Standard-MED ist eine erythemal wirksame Strahlungsdosis von 250 $J/m^2$ definiert.

Ein Verfahren zur Bestimmung der Photostabilität beschreiben Berset et al. im International Journal of Cosmetic Science 1996, Seite 167-177. Mit dem Erhalt von mehr als 90 % der Absorption im Absorptionsmaximum nach sonnensimulierter Bestrahlung (5 und 10 MED) wird z. B. 2-Phenylbenzimidazol 5-Sulfonsäure als photostabil eingestuft.

Ein photostabilisierter UV-A-Filter ist ein Filter, der zwar allein unter Bestrahlung seine Absorptionsfähigkeit weitgehend oder völlig verliert, jedoch in Kombination mit geeigneten weiteren Substanzen seine Absorptionsfähigkeit unter Bestrahlung weitgehend erhält.

Ein Beispiel für ein photostabile UV-A-Filter ist 2-(4-Diethylamino-2-hydroxybenzoyl)-benzoesäurehexylester (Uvinul®A Plus).

Unter dem Begriff UV-B-Filter werden öllösliche, wasserlösliche, schwerlösliche oder pigmentartige Substanzen verstanden, deren Absorptionsmaximum im UV-B-Bereich liegt, also im Bereich zwischen 290 und 320 nm.

Unter dem Begriff Breitbandfilter werden organische oder anorganische öllösliche, wasserlösliche, schwerlösliche oder pigmentartige Substanzen verstanden, die eine ausgeprägte Absorption sowohl im UV-A- als auch im UV-B-Bereich aufweisen, oder eine weitgehend konstante Absorption die gleichmässig vom UV-B Bereich in den UV-A Bereich übergeht.

Beispiele für öllösliche UV-B-Filter sind:

3-Benzylidencampher-Derivate, vorzugsweise 3-(4-Methylbenzyliden)campher, 4-Aminobenzoesäure-Derivate, vorzugsweise 4-(Dimethylamino)-benzoesäure(2-ethylhexyl)ester, 4-(Dimethylamino)benzoesäureamylester, 4-Bis (polyethoxy)aminobenzoesäurepolyethoxyethylester (unter der Handelsbezeichnung Uvinul® P25 von der Fa. BASF AG erhältlich);

sowie an Polymere gebundene UV-B-Filter (z.B. Benzyliden Malonat Polysiloxan, INCI: Polysilicone-15).

Beispiel für einen erfindungsgemäß vorteilhaften wasserlöslichen UV-B Filter ist 2-Phenylbenzimidazol-5-sulfonsäure und deren Natrium-, Kalium- oder Triethanolammoniumsalze.

Beispiele für bei Raumtemperatur flüssige UV-B-Filtersubstanzen im Sinne der vorliegenden Erfindung sind Homomenthylsalicylat (Homosalat), Ethylhexylsalicylat, 2-Ethylhexyl-2-cyano-3,3-diphenylacrylat (Octocrylen), 2-Ethylhexyl-2-hydroxybenzoat, Ester der Zimtsäure, vorzugsweise 4-Methoxyzimtsäure(2-ethylhexyl)ester (Ethylhexyl Methoxycinnamat) und 4-Methoxyzimtsäureisopentylester und (3-(4-(2,2-bis-Ethoxycarbonylvinyl)-phenoxy)propenyl)-methylsiloxan / Dimethylsiloxan Copolymer, welches beispielsweise bei DSM unter der Handelsbezeichnung Parsol® SLX erhältlich ist.

Beispiele für UV-Breitband-Filter sind Derivate des Benzophenons, vorzugsweise 2-Hydroxy-4-methoxybenzophenon (unter der Handelsbezeichnung Uvinul® M40 von der Fa. BASF AG erhältlich), 2-Hydroxy-4-methoxy-4'-methylbenzophenon, 2,2'-Dihydroxy-4-methoxybenzophenon, 2-Hydroxy-4-methoxybenzophenon-5-sulfonsäure (unter der Handelsbezeichnung Uvinul® MS40 von der Fa. BASF AG erhältlich); Triazine wie Bis-Resorcinyltriazinderivate, insbesondere 2,4-Bis-{[4-(2-Ethyl-hexyloxy)-2-hydroxy]-phenyl}-6-(4-methoxyphenyl)-1,3,5-triazin, welches unter der Handelsbezeichnung Tinosorb® S bei der CIBA-Chemikalien GmbH erhältlich ist, Benzotriazole wie 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist, 2-(2H-benzotriazol-2-yl)-4-methyl-6-[2-methyl-3-[1,3,3,3-tetramethyl-1-[(trimethylsilyl)oxy]disiloxanyl]-propyl]-phenol (CAS-Nr.: 155633-54-8) mit der INCI-Bezeichnung Drometrizole Trisiloxane, welches von Fa. Chimex unter der Marke Mexoryl® XL hergestellt wird, sowie [2,4'-Dihydroxy-3-(2H-benzotriazol-2-yl)-5-(1,1,3,3-tetramethylbutyl)-2'-n-octoxy-5'-benzoyl]diphenylmethan, 2,2' Methylen-bis-[6-(2H-benzotriazol-2-yl)-4-(methyl)phenol], 2,2'-Methylen-bis-[6-(2H-benzotriazol-2-yl)-4-(1,1,3,3-tetramethylbutyl)phenol], 2-(2'-Hydroxy-5'-octylphenyl)-benzotriazol, 2-(2'-Hydroxy-3',5'-di-t-amylphenyl)benzotriazol und 2-(2'-Hydroxy-5'-methylphenyl)benzotriazol.

Weitere UV-Breitbandfilter Substanzen sind ferner anorganische Pigmente auf Basis von Metalloxiden und/oder anderen in Wasser schwerlöslichen oder unlöslichen Metallverbindungen, insbesondere auf Basis der Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$) oder Cers (z. B. $Ce_2O_3$), Mischoxiden der entsprechenden Metalle, Abmischungen aus solchen Oxiden sowie Bariumsulfat. Diese Pigmente sind röntgenamorph oder nicht-röntgenamorph. Besonders bevorzugt handelt es sich um Pigmente auf der Basis von ZnO oder $TiO_2$, insbesondere $TiO_2$.

Die Pigmente können vorteilhaft auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die als Breitbandfilter eingesetzten Pigmente können vorteilhaft oberflächlich behandelt (coated) sein, wobei

beispielsweise ein hydrophiler, amphiphiler oder hydrophober Charakter gebildet wird bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können auch Wasser enthalten.

Anorganische Oberflächenbeschichtungen im Sinne der vorliegenden Erfindung bestehen beispielsweise aus Aluminiumoxid ($Al_2O_3$), Aluminiumhydroxid $Al(OH)_3$, bzw. Aluminiumoxidhydrat, Natriumhexametaphosphat $(NaPO_3)_6$, Natriummetaphosphat $(NaPO_3)_n$, Siliciumdioxid ($SiO_2$) oder Eisenoxid ($Fe_2O_3$). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung bestehen beispielsweise aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel), Tri(m)ethoxycaprylylsilane, Diphenyl Capryl Methicone oder Alginsäure. Diese organischen Oberflächenbeschichtungen können allein, in Kombination und/oder in Kombination mit anorganischen Beschichtungsmaterialien vorkommen.

Als UV-Breitbandfilter geeignete oberflächenmodifizierte Zinkoxidpartikel und Titandioxidpartikel sind kommerziell erhältlich z. B. unter dem Namen Z-COTE® HP1, Z-COTE® MAX oder T-Lite™ SF, T-Lite™ SF-S oder T-Lite™ Max von der Firma BASF AG.

Beispiele für zugelassene kommerzielle UV-B-Filter sind 4-Aminobenzoesäure, N,N,N-Trimethyl-4(2-oxoborn-3-ylidenmethyl)-aniliniummethylsulfat, Homosalate, 2-Phenylbenzimidazol-5-sulfonsäure und der Natrium-, Kalium- oder Triethanolammoniumsalze, Alpha-(2-oxoborn-3-yliden)-toluen-4-sulfonsäure und der Natrium-, Kalium- oder Triethanolammoniumsalze, 2-Cyano-3,3-diphenyl-acrylsäure-2-ethylhexylester (Octocrylen), Polyacrylamidomethyl Benzyliden Campher, Ethylhexyl-Methoxy-cinnamat, Ethoxyliertes Ethyl-4-aminobenzoat, Isopentyl-4-methoxycinnamat, 2,4,6-Trianilino-p-(carbon-2'-ethyl-hexyl-1'-oxi)-1,3,5-triazin (Ethylhexyl Triazon), Diethylhexyl Butamido Triazon, 3-(4'-Methylbenzyliden)-DL-campher, 3-Benzyliden-campher, 2-Ethylhexylsalicylat, 4-Dimethylamino-benzoesäure-2-ethylhexylester und Dimethicodiethylbenza-Imalonat.

Beispiele für zugelassene kommerzielle Breitband-Filter, sogenannte UV-AB-Filter sind Oxybenzon (Benzophenon-3), Drometrizol Trisiloxan, 2-Hydroxy-4-methoxybenzophenon-5-sulfonsäure und deren Natriumsalz (Benzophenon-4), Methylen Bis-Benzotriazolyl Tetramethylbutylphenol, Bis-Ethylhexyloxyphenol Methoxyphenyl Triazin, Zinkoxid und Titandioxid.

Bevorzugt werden in dem erfindungsgemäßen Verfahren als UV-B-Filter und/oder Breitband-Filter, insbesondere als UV-B-Filter solche Substanzen eingesetzt, die eine hohe spezifische Absorption im UV-B-Bereich aufweisen, bevorzugt eine spezifische Absorption von A 1%/1cm (Absorption einer 1 % igen Lösung bei 1 cm Schichtdicke) von mindestens 800, insbesondere von mindestens 1000.

Beispiele für besonders geeignete hoch absorbierende UV-B-Filter sind Zimtsäurederivate wie Ethylhexyl Methoxycinnamat oder Isoamyl p-Methoxycinnamat, Campherderivate wie 3-(4'-Methylbenzyliden)-DL-campher, 2-Phenylbenzimidazol-5-sulfonsäure und deren Natrium-, Kalium- oder Triethanolammoniumsalze, oder Triazinderivate wie Ethylhexyl Triazon oder Diethylhexyl Butamido Triazon. Ganz besonders bevorzugte UV-B-Filter sind Triazinderivate wie Ethylhexyl Triazon oder Diethylhexyl Butamido Triazon.

Nach dem erfindungsgemäßen Verfahren enthält die hergestellte Zubereitung als UV-A-Filter 2-(4-Diethylamino-2-hydroxybenzoyl)-benzoesäurehexylester, und als UV-B-Filter mindestens eine hoch absorbierende Substanz ausgewählt aus Ethylhexyl Triazon oder Diethylhexyl Butamido Triazon.

Diese voranstehend genannten Zubereitungen können zusätzlich zu den UV-A- und UV-B-Filtersubstanzen noch Breitbandfilter wie Titandioxid und/oder Zinkoxid , bevorzugt Titandioxid, und/oder auch organische Breitbandfilter wie Methylen-Bis-Benzotriazolyl Tetramethylbutylphenol und/oder Bis-Ethylhexyloxyphenol Methoxyphenyl Triazin enthalten.

Diese Kombinationen von UV-A- und UV-B-Filtem weisen eine sehr gute Effizienz und Performance hinsichtlich des Verhältnisses von SPF zum Gewichtsanteil der eingesetzten UV-Filtersubstanz in der kosmetischen und/oder dermatologischen Zusammensetzung auf, d.h. bevorzugt ein Verhältnis von SPF Einheiten zum prozentualen Masseanteil an UV-Filtern von mindestens 1,5, bevorzugt von mindestens 2.

Das erfindungsgemäße Verfahren beinhaltet als UV-A-Filter 2-(4-Diethylamino-2-hydroxybenzoyl)benzoesäurehexylester und als UV-B-Filter 2,4,6-Trianilino-p-(carbon-2'-ethyl-hexyl-1'-oxi)-1,3,5-triazin (Uvinul T150, BASF) oder Diethylhexyl Butamido Triazon, allein oder Mischungen der beiden UV-B Filter.

Das in dem erfindungsgemäßen Verfahren eingestellte Verhältnis der Summe der Masse der UV-A-Filter zur Summe der Masse der UV-B-Filter und Breitband-Filter von 1 bis 6 lässt sich auch durch folgende Formel darstellen:

$$1 \leq \Sigma (m_{UV\text{-}A}) / [\Sigma (m_{UV\text{-}B}) + \Sigma (m_{UV\text{-}Breitband})] \leq 6,$$

wobei

$m_{UV-A}$ Masse an einzelnem UV-A-Filter,
$m_{UV-B}$ Masse an einzelnem UV-B-Filter, und
$m_{UV-Breitband}$ Masse an einzelnem Breitband-Filter bedeutet.

Die Masse des UV-A-Filters in der Formulierung beträgt üblicherweise bis zu 30 Gew.-%, bevorzugt bis zu 25 Gew.-%. Insbesondere liegt sie in einem Bereich von 0,5 Gew.-% bis 15 Gew.-%.

Die Summe der Masse aller UV-B-Filter und Breitbandfilter in der Formulierung beträgt üblicherweise bis zu 40 Gew.-%, bevorzugt bis zu 25 Gew.-%. Insbesondere liegt sie in einem Bereich von 0,5 Gew.-% bis 20 Gew.-%.

Die nach dem erfindungsgemäßen Verfahren herstellbaren kosmetischen und/oder dermatologischen Zubereitungen sind in der Regel auf der Basis eines Trägers, der mindestens eine Ölphase enthält, aufgebaut. Es sind aber auch Zubereitungen allein auf wässriger Basis bei Verwendung von Verbindungen mit hydrophilen Substituenten möglich. Dem gemäß kommen Öle, Öl-in-Wasser- und Wasser-in-Öl-Emulsionen, Cremes und Pasten, Lippen- und Hautschutzstiftmassen oder fettfreie Gele, sowie Sprays und Schäume in Betracht.

Als Emulsionen kommen u.a. auch O/W-Makroemulsionen, O/W-Mikroemulsionen oder O/W/O-Emulsionen in Frage, wobei die Emulsionen durch Phaseninversionstechnologie, gemäß DE-A-197 26 121 erhältlich sind.

Übliche kosmetische Hilfsstoffe, die als Zusätze in Betracht kommen können, sind z. B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O-und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium-und/oder Zinkstearateingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Ein zusätzlicher Gehalt an Antioxidantien ist im allgemeinen bevorzugt. So können als günstige Antioxidantien alle für kosmetische und/oder dermatologische Anwendungen geeigneten oder gebräuchlichen Antioxidantien verwendet werden.

Vorteilhafterweise werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. ß-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximine, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirurin, Biliverdin, EDTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und deren Derivate (z.B. Ascorbylpalmitat, Mg-Ascorbylphosphat, Ascorbylacetat), Tocopherol und Derivate (z.B. Vitamin-E-Acetat, Tocotrienol), Vitamin A und Derivate (Vitamin-A-Palmitat) sowie Koniferylbenzoat des Benzoeharzes, Rutinsäure und deren Derivate, $\alpha$-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$), Selen und dessen Derivate (z.B.

Selenmethionin), Stilbene und deren Derivate (z.B. Stilbenoxid, Trans-Stilbenoxid).

Ferner vorteilhaft sind natürliche pflanzliche Antioxydantienkomplexe wie zum Beispiel Tee-, Trauben-oder Algen-Extrakte, aber auch natürliche oder naturidentische Einzelsubstanzen, wie z. B. Resveratrol.

Antioxydantien können neben dem Schutz des kosmetischen und/oder dermatologischen Produktes vor Oxidation auch anti-oxidative wie auch anti-aging Wirkungen in der menschlichen Haut erzielen.

Ganz besonders bevorzugt im Sinne der Erfindung sind daher Antioxidantien, die in die menschliche Haut penetrieren und dort effizient ihre Wirkung entfalten, und damit in gewissem Sinne synergistisch zu den Lichtschutzfiltem die Haut vor den Schäden des UV-Lichtes, vor Sonnenbrand und vor reaktiven Sauerstoffspezies und freien Radikalen schützen. Ganz besonders bevorzugt sind Vitamin C und Vitamin E und ihre Derivate.

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate als Antioxidans verwendet werden, ist es vorteilhaft, deren jeweilige Konzentration aus dem Bereich von 0,001 bis 10 Gew.-%, bezogen auf das Gesamtgewicht der Formulierung, zu wählen.

Sofern Vitamin A und/oder dessen Derivate bzw. Carotinoide das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure. Diese Aufzählung ist jedoch beispielhaft und nicht umfassend.

Den Zubereitungen im Sinne der vorliegenden Erfindung können ferner natürliche und/oder naturidentische und/oder synthetische Wirksubstanzen mit unterschiedlichen Wirkfunktionen zugesetzt sein, wie zum Beispiel Coffein zur Hautstraffung oder Durchblutungsförderung, Dihydroxyaceton und/oder Erythrulose zum Zwecke der Selbstbräunung, Bisabolol und/oder Panthenol zur Hautberuhigung und/oder Stoffe zur Feuchtigkeitsanreicherung (Moisturizing), zur Hautglättung, und insbesondere Wirksubstanzen zum Schutz vor Hautalterung, wie zum Beispiel Vitamin A und/oder dessen Derivate, Pflanzenextrakte oder auch proteinartige Substanzen.

Weitere Komponenten kosmetischer und /oder dermatologischer Zubereitungen im Sinne der vorliegenden Erfindungen können zusätzliche Funktionen erfüllen, wie z. B. die Färbung der Haut in der dekorativen Kosmetik, aber auch die des Produktes selbst. Hier werden in der Regel pigmentartige, öllösliche und/oder wasserlösliche kosmetische farbgebende Rohstoffe verwendet.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Die nach dem erfindungsgemäßen Verfahren erhältlichen Zusammensetzungen sind also insbesondere Sonnenschutzpräparate, die in flüssiger, pastöser oder fester Form vorliegen können, beispielsweise als Wasser-in-Öl-Cremes, Öl-in-Wasser-Cremes oder -Lotionen, Aerosol- und Pumpsschäume, Schaumcremes, Gele, Öle, Fettstifte, Puder, Sprays oder alkoholisch-wässrige Lotionen.

Schließlich können weitere an sich bekannte im UV-Bereich absorbierende Substanzen mitverwendet werden, sofern sie im Gesamtsystem der zu verwendenden Kombination aus UV-Filtern stabil sind.

Bevorzugte Ausführungsformen der verschiedenen übrigen Parameter und Komponenten wurden bereits im vorangehenden Teil der Beschreibung angegeben.

Bevorzugte UV-B-Filter wurden bereits voranstehend im Zusammenhang mit der Beschreibung des erfindungsgemäßen Verfahrens zur Erhöhung des Lichtschutzfaktors einer kosmetische und/oder dermatologische Zubereitung erläutert.

Ebenfalls wurde voranstehend erläutert, welche Verbindungen als Breitbandfilter definiert sind.

Die Erfindung wird durch folgende, die Erfindung jedoch nicht einschränkende Beispiele erläutert.

Beispiel 1

Die Komponenten der Phase A (siehe Tabelle 1) wurden auf 80°C erwärmt. Die Komponenten der Phase B (siehe Tabelle 1) wurden ebenfalls auf ca. 80°C erwärmt und unter Homogenisieren in die 80°C heiße Phase A eingerührt. Das Gemisch der beiden Phasen wurde unter Rühren auf ca. 40°C abgekühlt. Bei ca. 40°C wurde Phase C (siehe Tabelle 1) hinzugegeben. Das Gemisch wurde nochmals kurzzeitig homogenisiert und anschließend ließ man es unter Rühren auf Raumtemperatur abkühlen.

Tabelle 1

| Phase | Tradename | | Gew.-[%] |
|---|---|---|---|
| | | | |
| A | Uvinul® T 150 | Ethylhexyl Triazone | 2,00 |
| | Uvinul® A Plus | Diethylamino Hydroxybenzoyl Hexyl Benzoate | 2,50 |
| | Cetiol B | Dibutyl Adipate | 8,00 |
| | Finsolv TN | $C_{12}$-$C_{15}$ Alkyl Benzoate | 8,00 |
| | Myritol 331 | Cocoglycerides | 12,00 |
| | Lanette E | Sodium Cetearyl Sulfate | 1,00 |
| | Eumulgin VL 75 | Lauryl Glucoside, Polyglyceryl-2 Dipolyhydroxystearate, Glycerin | 4,00 |
| | Lanette O | Cetearyl Alcohol | 2,00 |
| | | | |
| B | Edeta BD | Disodium EDTA | 0,05 |
| | Glycerin 87% | Glycerin | 3,00 |
| | Keltrol | Xanthan Gum | 0,30 |
| | Veegum Ultra | Magnesium Aluminum Silicate | 1,50 |
| | Water dem | Aqua dem | ad 99,00 |
| | | | |
| C | Euxyl K 300 | Phenoxyethanol, Methylparaben, Butylparaben, Ethylparaben, Propylparaben, Isobutylparaben | 1,00 |

Beispiele 2 bis 20 sowie Vergleichsbeispiele V1 und V2

Die Herstellung der folgenden in Tabelle 2 wiedergegebenen Beispiele erfolgte analog wie in Beispiel 1.

Tabelle 2

| Beispiel | 1 | 2 | 3 | 4 | 5 | 6 | 7 | V1 | 8* | V2 | 10* | 11* | 12 | 15 | 16 | 17 | 18 | 19 | 20 |
|---|---|---|---|---|---|---|---|---|---|---|---|---|---|---|---|---|---|---|---|
| | | | | | | | | | | | | | | | | | | | |
| Uvinul®→ A Plus | 2,5 | 5 | 10 | 2,5 | 7,5 | 2 | 10 | 2 | 10 | | 3.5 | 10 | 3.5 | 3,5 | 7 | 7 | 4 | 10 | 3.5 |
| BMDBM | | | | | | | | | | 2 | | | | | | | | | |
| Ethylhexyl Triazon | 2 | 2 | 2 | 2,5 | 2,5 | | | | | | | | 2.5 | 2 | 2 | 2 | 2.2 | 2,5 | 2 |
| Diethylhexyl Butamido Triazon | | | | | | 2 | 2 | | | | | | | | | | | | |
| Octocryfen | | | | | | | | 5 | 5 | 5 | | | | | | | | | |
| Tinosorb S | | | | | | | | | | | 2 | 2 | | | | | | | |
| $TiO_2$ | | | | | | | | | | | | | | | | | 2 | | 1,5 |
| ZnO | | | | | | | | | | | | | | | | 3,5 | | 3 | |
| Ratio Σ UV-A/Σ (UV-B+UV-AB) | 1,25 | 2,5 | | 1 | 3 | 1 | 5 | 0,4 | 2 | 0,4 | 1,75 | 5 | 1,4 | 1,75 | 3,5 | 1,27 | 0,95 | 1,82 | 1 |
| SPF in vivo | 10 | 20 | 32 | 23 | 30 | 15 | 32 | 15 | 32 | 16 | 27<br>13 | 19 | 20 | 14 | 19 | 28 | 30 | 43 | 19 |

* vergleichendes Beispiel

Die Zahlenangaben der verschiedenen UV-Filter-Substanzen sind Gew.-% Angaben.

## Patentansprüche

1. Verfahren zur Erhöhung des Lichtschutzfaktors einer kosmetischen und/oder dermatologischen Zubereitung, die mindestens einen UV-B-Filter, ausgewählt aus Ethylhexyl Triazon und Diethylhexyl Butamido Triazon, und, optional UV-Breitband-Filter enthält, durch Zugabe eines UV-A-Filters während der Herstellung der Zubereitung, **dadurch gekennzeichnet, dass** in der fertigen kosmetischen und/oder dermatologischen Zubereitung ein Wert für das Verhältnis der Summe der Masse der UV-A-Filter zur Summe der Masse der UV-B-Filter und Breitband-Filter von 1 bis 6 eingestellt wird, **dadurch gekennzeichnet, dass** 2-(4-Diethylamino-2-hydroxybenzoyl)-benzoesäurehexylester als einziger UV-A-Filter eingesetzt wird.

2. Verwendung des UV-A-Filters 2-(4-Diethylamino-2-hydroxybenzoyl)-benzoesäurehexylester zur Erhöhung des Lichtschutzfaktors einer kosmetischen und/oder dermatologischen Zubereitung, die mindestens einen UV-B- Filter, ausgewählt aus Ethylhexyl Triazon und Diethylhexyl Butamido Triazon, und, optional UV-Breitband-Filter enthält, **dadurch gekennzeichnet, dass** in der fertigen kosmetischen und/oder dermatologischen Zubereitung der Wert für das Verhältnis des UV-A-Filters zur Summe der Masse der UV-B-Filter und Breitband-Filter 1 bis 6 beträgt, **dadurch gekennzeichnet, dass** die Zubereitung 2-(4-Diethylamino-2-hydroxybenzoyl)-benzoesäurehexylester als einzigen UV-A-Filter enthält.

3. Lichtschutzwirksame kosmetische und/oder dermatologische Zubereitung, enthaltend als einzigen UV-A-Filter 2-(4-Diethylamino-2-hydroxybenzoyl)-benzoesäurehexylester und mindestens einen UV-B-Filter, ausgewählt aus Ethylhexyl Triazon und Diethylhexyl Butamido Triazon, sowie gegebenenfalls weitere kosmetische Wirk-, Hilfs- und Zusatzstoffe, wobei der Wert für das Verhältnis der Summe der Masse der UV-A-Filter zur Summe der Masse der UV-B-Filter 1 bis 8 beträgt, und die Zubereitung keinen Breitband-Filter enthält.

## Claims

1. A method for increasing the sun protection factor of a cosmetic and/or dermatological preparation which comprises at least one UV-B filter selected from ethylhexyl triazone and diethylhexyl butamido triazone, and, optionally, a UV broadband filter by adding a UV-A filter during the production of the preparation, wherein, in the finished cosmetic and/or dermatological preparation, a value for the ratio of the sum of the mass of the UV-A filters to the sum of the mass of the UV-B filters and broadband filters of from 1 to 6 is established, wherein hexyl 2-(4-diethylamino-2-hydroxybenzoyl) benzoate is used as the sole UV-A filter.

2. The use of the UV-A filter hexyl 2-(4-diethylamino-2-hydroxybenzoyl)benzoate for increasing the sun protection factor of a cosmetic and/or dermatological preparation which comprises at least one UV-B filter selected from ethylhexyl triazone and diethylhexyl butamido triazone, and, optionally, a UV broadband filter, wherein, in the finished cosmetic and/or dermatological preparation, the value for the ratio of the UV-A filter to the sum of the mass of the UV-B filters and broadband filters is from 1 to 6, wherein the preparation comprises hexyl 2-(4-diethylamino-2-hydroxybenzoyl)benzoate as the sole UV-A filter.

3. A photoprotective cosmetic and/or dermatological preparation comprising hexyl 2-(4-diethylamino-2-hydroxybenzoyl)benzoate as the sole UV-A filter and at least one UV-B filter selected from ethylhexyl triazone and diethylhexyl butamido triazone, and optionally further cosmetic active ingredients, auxiliaries and additives, where the value for the ratio of the sum of the mass of the UV-A filters to the sum of the mass of the UV-B filters is from 1 to 8, and the preparation comprises no broadband filter.

## Revendications

1. Procédé pour augmenter le facteur photoprotecteur d'une préparation cosmétique et/ou dermatologique, qui contient au moins un filtre UV-B, choisi parmi Ethylhexyl Triazone et Diethylhexyl Butamido Triazone et, éventuellement, un filtre UV à large bande, par addition d'un filtre UV-A pendant la fabrication de la préparation, **caractérisé en ce que** dans la préparation cosmétique et/ou dermatologique finale on ajuste à une valeur de 1 à 6 le rapport de la somme de la masse des filtres UV-A sur la somme de la masse des filtres UV-B et des filtres à large bande, **caractérisé**

**en ce qu'**on utilise comme unique filtre UV-A du 2-(4-diéthylamino-2-hydroxybenzoyl)-benzoate d'hexyle.

2. Utilisation du filtre UV-A 2-(4-diéthylamino-2-hydroxybenzoyl)-benzoate d'hexyle pour augmenter le facteur photoprotecteur d'une préparation cosmétique et/ou dermatologique, qui contient au moins un filtre UV-B choisi parmi Ethylhexyl Triazone et Diethylhexyl Butamido Triazone et, éventuellement, un filtre UV à large bande, **caractérisée en ce que** dans la préparation cosmétique et/ou dermatologique finale la valeur du rapport du filtre UV-A sur la somme de la masse des filtres UV-B et filtres à large bande est de 1 à 6, **caractérisée en ce que** la préparation contient comme unique filtre UV-A du 2-(4-diéthylamino-2-hydroxybenzoyl)-benzoate d'hexyle.

3. Préparation cosmétique et/ou dermatologique à action photoprotectrice, contenant du 2-(4-diéthylamino-2-hydroxybenzoyl)-benzoate d'hexyle comme unique filtre UV-A et au moins un filtre UV-B, choisi parmi Ethylhexyl Triazone et Diethylhexyl Butamido Triazone, ainsi qu'éventuellement d'autres agents actifs, adjuvants et additifs cosmétiques, la valeur du rapport de la somme de la masse des filtres UV-A sur la somme de la masse des filtres UV-B étant de 1 à 8, et la préparation ne contenant pas de filtres à large bande.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1046391 A
- WO 2005094772 A
- EP 1290999 A
- WO 03039507 A
- DE 19726121 A


### In der Beschreibung aufgeführte Nicht-Patentliteratur


- *Chemie in unserer Zeit,* 2004, vol. 38, 98-112
- Measurement standard for UVA protection efficacy. Japan Cosmetic Industry Association (JCIA), 01. Januar 1996
- *Int. J. Cosm. Science,* 1988, vol. 10, 53
- *Proceedings zum 21st IFSCC International Congress,* 2000, 530
- **SCHRADER ; GRUNDLAGEN ; REZEPTUREN.** Handbüchern der Kosmetik. Hüthig Verlag, 1989
- **UMBACH.** Kosmetik: Entwicklung, Herstellung und Anwendung kosmetischer Mittel, 2. Georg Thieme Verlag, 1995
- **BERSET et al.** *International Journal of Cosmetic Science,* 1996, 167-177
- *CHEMICAL ABSTRACTS,* 155633-54-8